# EUROPEAN PATENT SPECIFICATION

(11) **EP 3 336 548 B1**
(45) Date of publication and mention of the grant of the patent: **14.04.2021**
(21) Application number: 16835483.5
(22) Date of filing: 12.08.2016
(51) Int. Cl.: G01N 33/574, C12Q 1/68, A61K 31/7105, C12Q 1/6883, A61P 35/02

(54) **METHOD FOR PROVIDING INFORMATION ON CHRONIC MYELOID LEUKEMIA**
VERFAHREN ZUR BEREITSTELLUNG VON INFORMATIONEN ÜBER CHRONISCHE MYELOISCHE LEUKÄMIE
PROCÉDÉ DE FOURNITURE D'INFORMATIONS SUR LA LEUCÉMIE MYÉLOÏDE CHRONIQUE

(30) Priority: 12.08.2015 KR 20150113704
(43) Date of publication of application: 20.06.2018
(73) Proprietor: The Catholic University Of Korea Industry-Academic Cooperation Foundation, Seocho-gu Seoul 06591 (KR); Research Business Foundation Sungkyunkwan University, Suwon-si, Gyeonggi-do 16419 (KR)
(72) Inventor: KIM, Dong Wook, Seoul 07345 (KR); KIM, Soo Hyun, Seoul 08275 (KR); KIM, Hong Tae, Anyang-si Gyeonggi-do 14101 (KR); HAN, Seung Hun, Suwon-si Gyeonggi-do 16324 (KR)
(74) Representative: Schnappauf, Georg
(86) International application number: PCT/KR2016/008897
(87) International publication number: WO 2017/026843

(56) References cited:
- WO-A1-2010/007464
- WO-A2-2009/013614
- WO-A2-2010/132861
- KR-A- 20090 078 349
- KR-A- 20120 095 722
- US-A1- 2011 190 157
- US-A1- 2012 171 670
- ZHIGANG WANG ET AL: "Identification of a 5-gene signature for clinical and prognostic prediction in gastric cancer patients upon microarray data", MEDICAL ONCOLOGY, vol. 30, no. 3, 3 August 2013 (2013-08-03) , XP055537986, GB ISSN: 1357-0560, DOI: 10.1007/s12032-013-0678-5
- DATABASE PROTEIN [Online] 28 July 2005 XP055514320 Retrieved from NCBI Database accession no. AAH71588
- RONCHETTI, DOMENICA ET AL.: 'Small Nucleolar RNAs as New Biomarkers in Chronic Lymphocytic Leuxemia' BMC MEDICAL GENOMICS vol. 6, no. 27, 2013, pages 1 - 11, XP021161966

## Description

The present teaching relates to a method for providing information on chronic myeloid leukemia

### Background Art

Leukemia refers to all diseases in which leukocytes proliferate neoplastically. Leukemia maybe classified into myeloid leukemia and lymphocytic leukemia based on the leukocytes from which the leukemia originates, and also be classified into acute leukemia and chronic leukemia based on the rate of progress. The clinical patterns of leukemia vary depending on the type of disease and the characteristics of the invaded cells. Lymphocytic leukemia is caused by the mutation of lymphocytic blood cells, myeloid leukemia is caused by the mutation of myeloid blood cells, and chronic myeloid leukemia (CML) is caused by malignant clones of pluripotent hematopoietic stem cells with mutations.

Leukemia refers to all diseases in which leukocytes proliferate neoplastically. Leukemia may be classified into myeloid leukemia and lymphocytic leukemia based on the leukocytes from which the leukemia originates, and also be classified into acute leukemia and chronic leukemia based on the rate of progress. The clinical patterns of leukemia vary depending on the type of disease and the characteristics of the invaded cells. Lymphocytic leukemia is caused by the mutation of lymphocytic blood cells, myeloid leukemia is caused by the mutation of myeloid blood cells, and chronic myeloid leukemia (CML) is caused by malignant clones of pluripotent hematopoietic stem cells with mutations.

In particular, chronic myeloid leukemia (CML) is a malignant blood cancer in which a large amount of hematopoietic stem cells proliferate in bone marrow and a variety of abnormally increased blood cells invade peripheral blood or other organs, and which occurs mainly in adults. The most common method for diagnosing chronic myeloid leukemia is to detect the Philadelphia chromosome by a chromosome test. In fact, a reciprocal translocation between translocation of chromosome 9 and chromosome 22 is found in about 95% of chronic myeloid leukemia patients. In this translocation, the Philadelphia chromosome is formed by translocation of a portion of the abelson oncogene (abl) gene located at q34 on chromosome 9 to the breakpoint cluster region (bcr) gene located at q11.2 on chromosome 22. This rearrangement is called BCR-ABL1 rearrangement (Korean Patent Application No. 10-2012-002466). For this reason, many studies have been actively conducted to detect the BCR-ABL rearrangement in older to diagnose chronic myeloid leukemia and predict the prognosis of chronic myeloid leukemia. However, leukemias without mutation in the BCR-ABL1 gene have frequently occurred. Particularly, there have been little or no studies on the mechanism by which chronic myeloid leukemia transforms into acute myeloid leukemia (AML) and acute lymphoid leukemia (ALL) and on methods for diagnosing them.

Furthermore, in the past, the primary treatment for chronic myeloid leukemia was hematopoietic stem cell transplantation, but in recent years, a method of inhibiting the BCR-ABL1 oncogenic protein using various tyrosine kinase inhibitors (TKIs) has become a common treatment method. However, some patients will have resistance to the tyrosine kinase inhibitors at the beginning or during the treatment process. In this regard, it is known that mutations in the BCR-ABL1 kinase domain play an important role in resistance to the tyrosine kinase inhibitors. However, such mutations are not observed in some patients, and the mechanism of the resistance is not still well known.

Accordingly, there is a need to develop methods capable of diagnosing chronic myeloid leukemia independently of BCR-ABL1 and predicting the progression, drug resistance and prediction of chronic myeloid leukemia.

### Technical Problem

The present teaching has been made in order to solve the above-described problems occurring in the art, and it is an object of the present teaching to provide a method of providing information, such as diagnosis of the transformation of chronic myeloid leukemia to an acute phase, prediction of prognosis after the transformation of chronic myeloid leukemia to an acute phase, resistance to tyrosine kinase inhibitors, etc., by use of Cordon-bleu protein-like 1 (Coblll).

However, objects which are to be achieved by the present teaching are not limited to the above-mentioned objects, and other objects of the present teaching will be clearly understood by those skilled in the art from the following description.

### Technical Solution

Hereinafter, various embodiments described herein will be described with reference to figures. In the following description, numerous specific details are set forth, such as specific configurations, compositions, and processes, etc., in order to provide a thorough understanding of the present teaching. However, certain embodiments may be practiced without one or more of these specific details, or in combination with other known methods and configurations. In other instances, known processes and preparation techniques have not been described in particular detail in order to not unnecessarily obscure the present teaching. Reference throughout this specification to "one embodiment" or "an embodiment" means that a particular feature, configuration, composition, or characteristic described in connection with the embodiment is included in at least one embodiment of the present teaching. Thus, the appearances of the phrase "in one embodiment" or "an embodiment" in various places throughout this specification are not necessarily referring to the same embodiment of the present teaching. Additionally, the particular features, configurations, compositions, or characteristics may be combined in any suitable manner in one or more embodiments.

As used herein, "biological sample" refers to any sample that may be used to analyze the expression level of the Coblll gene *in vivo.* Preferably, the biological sample may be blood, plasma, serum, bone marrow, tissue, cells, saliva, sputum, hair, urine, or the like. More preferably, the biological sample may be blood, bone marrow cells or the like. However, the biological sample is not limited thereto and maybe any sample that may be used to measure expression of Coblll protein or Coblll gene such as Coblll RNA or the like.

As used herein, "expression level measurement" refers to a process that determines the presence and/or expression level of an mRNA or protein encoded by a marker gene in a biological sample in order to confirm information on chronic myeloid leukemia The expression level measurement may be performed by either measuring the level of mRNA by use of a primer set or probe set that binds to the mRNA of a marker gene or by measuring the level of protein by use of a binding molecule that binds specifically to the marker gene protein. Analysis methods for expression level measurement include RT-PCR, competitive RT-PCR, real-time RT-PCR, RNase protection assay (RPA), Northern blotting, DNA microarray chip assay, Western blotting, enzyme linked immunosorbent assay (ELISA), radioimmunoassay, radioimmunodifiusion, Ouchterlony immunodiffusion, Rocket immunoelectrophoresis, immunohistochemical staining, immunoprecipitation assay, complete fixation assay, FACS, protein chip assay, and the like. However, the analysis methods are not limited thereto and may include any methods that may be used to measure the marker gene mRNA or marker gene protein level.

As used herein, "kit" refers to a testing device capable of detecting and/or measuring expression of the Cobll1 gene in order to confirm information on chronic myeloid leukemia. The kit may be any kit capable of detecting expression of Cobll1 in a biological sample isolated from a patient. Preferably, the kit may comprise a probe or primer set having a sequence complementary to Cobll1 mRNA, or may comprise an antibody, an aptamer, a ligand or the like, which binds specifically to the Cobll1 protein. However, the kit is not limited thereto and may be any other kit capable of detecting and/or measuring expression of the Cobll1 gene.

As used herein, "method of providing information" refers to a method of providing information on chronic myeloid leukemia by measuring the expression level of Cordon-bleu protein-like 1 (Cobll1) in a biological sample isolated from a patient Preferably, the method is a method of providing information on chronic myeloid leukemia, such as whether chronic myeloid leukemia progressed to an acute phase, prediction of survival time after the transformation of chronic myeloid leukemia to an acute phase, whether chronic myeloid leukemia has resistance to tyrosine kinase inhibitor drugs, etc. However, the information is not limited thereto and may include any other information that may be confirmed by measuring the expression level of the Cobll1 gene.

The present teaching provides a method for providing information on the progression of chronic myeloid leukemia to an acute phase, the method comprising a step of measuring the expression level of Cordon-bleu protein-like 1 (Cobll1) in abiological sample isolated from a patient.

In an embodiment of the present teaching, the method for providing information on the progression of chronic myeloid leukemia to an acute phase may comprise diagnosing that chronic myeloid leukemia has progressed to the acute phase, when the expression level of Cobll1 increased compared to that in a normal control group.

The present teaching also provides a method for providing information on the prognosis of chronic myeloid leukemia, the method comprising a step of measuring the expression level of Cordon-bleu protein-like 1 (Cobll1) in a biological sample isolated from a patient

In an embodiment of the present teaching, the method for providing information on the prognosis of chronic myeloid leukemia may comprise predicting survival time after the transformation of chronic myeloid leukemia to an acute phase. Preferably, the method may comprise predicting that the survival time is short, when the expression level of Cobll1 increased compared to that in a normal control group.

The present teaching also provides a method for providing information on the resistance of a chronic myeloid leukemia patient to a tyrosine kinase inhibitor, the method comprising a step of measuring the expression level of Cordon-bleu protein-like 1 (Cobll1) in a biological sample isolated from a patient

In an embodiment of the present teaching, the method for providing information on the resistance may comprise predicting that the patient has resistance to the tyrosine kinase inhibitor, when the expression level of Cobll1 increased compared to that in a normal control group.

In another embodiment, the tyrosine kinase inhibitor may preferably be a drug, such as imatinib, nilotinib or the like, which may be used to treat chronic myeloid leukemia by inhibiting tyrosine kinase activity. However, it is not limited thereto and may also be applicable to any therapeutic method, such as immunotherapy, hematopoietic stem cell transplantation, which is used for treatment of chronic myeloid leukemia

In an embodiment of the present teaching, the biological sample may preferably be blood, plasma, serum, bone marrow, tissue, cells, saliva, sputum, hair, urine, or the like. More preferably, the biological sample may be blood, bone marrow cells or the like. However, the biological sample is not limited thereto and may be any sample that may be used to measure expression of Cobll1 protein or Coblll gene such as Cobll1 RNA or the like.

In other embodiments of the present teaching, the method for measuring the expression level of Cobll1 may preferably be radioimmunoassay, radioimmunoprecipitation assay, immunoprecipitation assay, ELISA (enzyme-linked immunosorbentassay), sandwich assay, real-time PCR, RT-PCR, Western blotting, or the like. However, the method is not limited thereto and may also be any method capable of measuring expression of Cobll1 such as Cobll1 protein, Cobll1 RNA or the like.

The present teaching also provides a composition for diagnosing the progression of chronic myeloid leukemia to an acute phase, the composition comprising a binding molecule specific for Coblll, a probe set complementary to Cobll1, or a primer set complementary to Cobll1.

The present teaching also provides a kit for diagnosing the progression of chronic myeloid leukemia to an acute phase, the kit comprising the composition for diagnosing.

In an embodiment of the present teaching, the binding molecule may preferably be an antibody, an aptamer, a ligand or the like. More preferably, the binding molecule may be an antibody produced using the amino acid sequence of SEQ ID NO: 2 as an antigen. However, the binding molecule is not limited thereto and may be any substance that may bind specifically to the Cobll1 protein.

The present teaching also provides a Cordon-bleu protein-like 1 (Cobll1) binding antibody produced using the amino acid sequence of SEQ ID NO: 2 as an antigen.

The present teaching also provides a pharmaceutical composition for treating chronic myeloid leukemia, comprising, as an active ingredient, an oligonucleotide that binds specifically to Cordon-bleu protein-like 1 (Cobll1) mRNA

In an embodiment of the present teaching, the oligonucleotide may preferably be siRNA (silencing RNA), miRNA (miRNA), shRNA (short hairpin RNA) or the like. More preferably, the oligonucleotide may be an siRNA comprising one or more of the nucleotide sequences of SEQ ID NOs: 3 to 6. However, it means binding to Cobll1 mRNA and preventing overexpression of the protein, and in a broad sense, includes all therapeutic methods that inhibit the function of the Coblll protein, thereby inhibiting the progression and drug resistance of chronic myeloid leukemia.

In another embodiment of the present teaching, the pharmaceutical composition may inhibit expression of the Cobll1 protein, thereby inhibiting the progression of chronic myeloid leukemia to an acute phase.

In another embodiment of the present teaching, the pharmaceutical composition may be used either to either inhibit expression of overexpressed Cobll1 protein, thereby returning chronic myeloid leukemia, which progressed to an acute phase, to a chronic phase, or inhibit the function of overexpressed Cobll1 protein, thereby returning chronic myeloid leukemia, which progressed to an acute phase, to a chronic phase.

In the present teaching, the pharmaceutical composition may be in the form of capsules, tablets, granules, injectable solutions, ointments, powders or beverages, and the pharmaceutical composition may be for administration to humans. For use, the pharmaceutical composition may be formulated as oral dosage forms, including powders, granules, capsules, tablets, aqueous suspensions and the like, external preparations, suppositories and sterile injectable solutions, according to conventional methods, but is not limited thereto. The pharmaceutical composition of the present teaching may comprise pharmaceutically acceptable carriers. Pharmaceutically acceptable carriers that may be used for oral administration include binders, lubricants, disintegrants, excipients, solubilizers, dispersing agents, stabilizers, suspending agents, pigments, fragrances, and the like. Pharmaceutically acceptable carriers that may be used for administration by injection include buffers, preservatives, analgesic agents, solubilizers, isotonic agents, stabilizers, and the like, and pharmaceutically acceptable carriers that may be used for topical administration include bases, excipients, lubricants, preservatives, and the like. The pharmaceutical composition of the present teaching may be formulated in various manners by mixing it with the pharmaceutically acceptable carriers as described above. For example, for oral administration, the pharmaceutical composition may be formulated in the form of tablets, troches, capsules, elixirs, suspensions, syrups, wafers, and the like, and for administration by injection, the pharmaceutical composition may be provided in the form of unit dosage ampoules or multiple dosage containers. In addition, the pharmaceutical composition may be formulated as solutions, suspensions, tablets, capsules, sustained-release preparations, and the like.

Meanwhile, examples of carriers, excipients and diluents suitable for formulation may include lactose, dextrose, sucrose, sorbitol, mannitol, xylitol, erythritol, maltitol, starch, gum acacia, alginate, gelatin, calcium phosphate, calcium silicate, cellulose, methylcellulose, microcrystalline cellulose, polyvinylpyrrolidone, water, methylhydroxy benzoate, propylhydroxy benzoate, talc, magnesium stearate, mineral oils or the like. In addition, the pharmaceutical composition may further comprise fillers, anti-coagulating agents, lubricants, wetting agents, fragrances, emulsifiers, preservatives or the like.

Routes for administration of the pharmaceutical composition according to the present teaching include, but are not limited to, oral, intravenous, intramuscular, intra-arterial, infra-marrow, intrathecal, intracardiac, transdermal, subcutaneous, intraperitoneal, intranasal, intestinal intrarectal, local, sublingual and intrarectal routes. Oral or parenteral administration is preferred. As used herein, the term "parenteral" is meant to include subcutaneous, intradermal, intravenous, intramuscular, intra-articular, intrabursal, intrathecal, intralesional and intracranial injection or infusion techniques. The pharmaceutical composition of the present teaching may also be administered in the form of suppositories for rectal administration.

The dose of the pharmaceutical composition of the present teaching may vary depending on the activity of a particular compound used, the patient's age, body weight, general health, sex, diet, administration time, the route of administration, excretion rate, drug combination, and the severity of a particular disease to be prevented or treated, and can be suitably determined by a person skilled in the art depending on the patient's condition, body weight, the severity of the disease, the form of drug, the route of administration, and the period of administration. The pharmaceutical composition may be administered at a dose of 0.0001-50 mg/kg/day or 0.001-50mg/kg/day. The pharmaceutical composition of the present teaching maybe administered once or several times a day. The dose does not limit the scope of the present teaching in any way. The pharmaceutical composition according to the present teaching may be formulated as pills, sugar-coated tablets, capsules, liquids, gels, syrups, slurries, or suspensions.

### Advantageous Effects

The method of providing information on chronic myeloid leukemia by use of Cordon-bleu protein-like 1 (Cobll1) according to the present teaching can simultaneously provide various information such as diagnosis of the transformation of chronic myeloid leukemia to an acute phase, prediction of prognosis after the transformation of chronic myeloid leukemia to an acute phase, resistance to tyrosine kinase inhibitors, etc., and may also be used to diagnose patients who could not be predicted and diagnosed by measurement of the expression level of the BCR-ABL1 gene according to the prior art. Thus, it is expected that the method of the present teaching can be used to diagnose various progressions of chronic myeloid leukemia and predict the drug resistance and prognosis of chronic myeloid leukemia. Furthermore, it is expected that the use of the oligonucleotide of the present teaching, which binds specifically to Cobll1 mRNA, can effectively inhibit expression of the Cobll1 protein so as to effectively inhibit cell proliferation and drug resistance, thereby treating chronic myeloid leukemia and effectively inhibiting the transformation of chronic myeloid leukemia to an acute phase. Thus, it is expected that the oligonucleotide of the present teaching may also be used for the development of a therapeutic method for inhibiting the function of the Cobll1 protein.

### Description of Drawings

FIG. 1 shows the results of identifying genes, which are involved in the progression of chronic myeloid leukemia to an acute phase, in one example of the present teaching.
FIG. 2 shows the results of further identifying genes, which are involved in the progression of chronic myeloid leukemia to an acute phase, in one example of the present teaching. In the graph of FIG. 2A, the first bar indicates CP1, and the second bar indicates BP.
FIG. 3 shows the production of an antibody against Cobll1 according to one example of the present teaching.
FIG. 4 shows the results of analyzing the correlation between the progression of chronic myeloid leukemia to an acute phase and the Coblll protein in one example of the present teaching.
FIG. 5 shows the results of analyzing the difference in expression of the Coblll protein between patients in one example of the present teaching.
FIG. 6 shows the results of analyzing the correlation between the amount of leukemic blast cells and the expression of the Cobll1 protein in one example of the present teaching.
FIG. 7 shows the results of analyzing the difference in expression level of the Cobll1 protein between various samples in one example of the present teaching.
FIG. 8 shows the results of analyzing the difference in expression level of the Cobll1 protein between the progression stages of chronic myeloid leukemia in one example of the present teaching.
FIG. 9 shows the results of analyzing the correlation between Cobll1 protein and BCR-ABL1 in one example of the present teaching.
FIG. 10 shows the results of analyzing the correlation between the Cob1 protein and the prognosis of chronic myeloid leukemia patients in one example of the present teaching.
FIG. 11 shows the results of analyzing expression of the Cobll1 protein in various types of cells in one example of the present teaching.
FIG. 12 shows the results of analyzing sensitivity to nilotinib in cells with inhibited expression of the Cobll1 protein in one example of the present teaching. In the graph of FIG. 12, the first, second and third bars indicate siControl, siCobll1-1 and sicobll-2, respectively.
FIG. 13 shows the results of sensitivity nilotinib in cells with increased expression of the Coblll protein in one example of the present teaching. In the graph of FIG. 13, the first bar indicates 0 nM, and the second bar indicates 10 nM.
FIG. 14 shows the results of evaluating the effect of the Coblll protein on cell proliferation in one example of the present teaching. In the graph ofFIG. 14A, the upper, middle and lower curves indicate Cobll1 (0 µg), Cobll1 (5 µg) and Coblll (15 µg). In the graph of FIG. 14B, the first bar indicates Mock, and the second bar indicates Coblll.
FIG. 15 shows the results of evaluating the effect of the Coblll protein on transformation into chronic myeloid leukemia in one example of the present teaching.

### Mode for Invention

Hereinafter, the present teaching will be described in further detail with reference to examples. It will be obvious to those skilled in the art that these examples are for illustrative purposes only and are not intended to limit the scope of the present teaching. The scope of the present teaching is defined and limited by the appended claims.

### Examples

### Example 1: Identification of Genes Involved in Progression of Chronic Myeloid Leukemia to Acute Phase

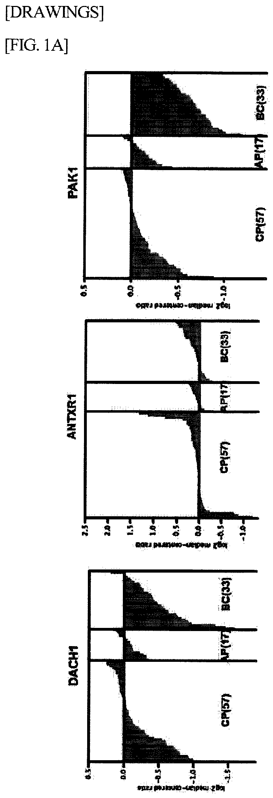

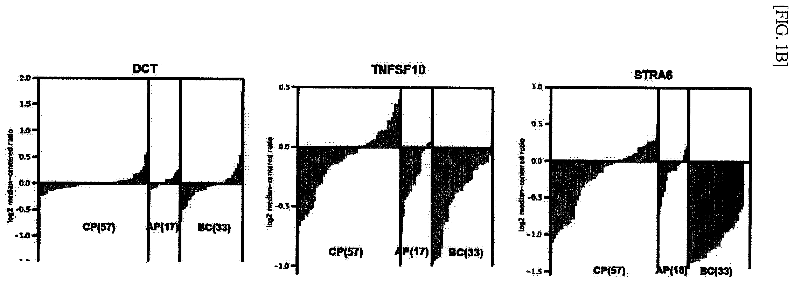

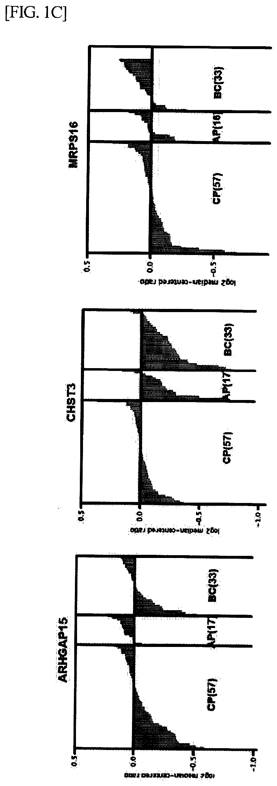

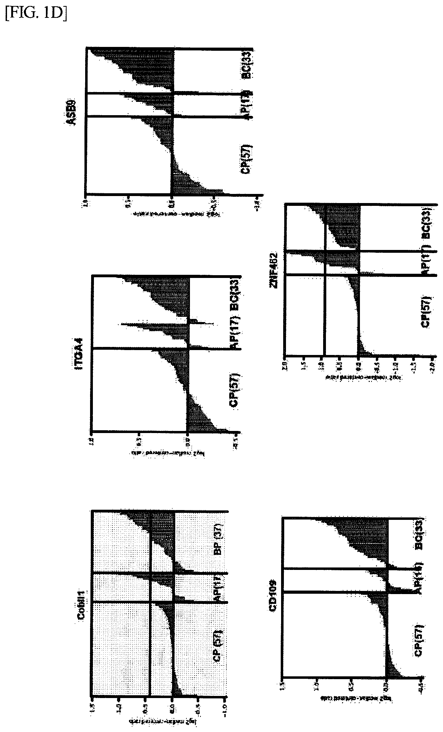

In order to identify genes, which are involved in drug resistance and the progression of chronic myeloid leukemia to an acute phase, independently of the BCR-ABL1 protein, gene expression in the K562 CML BP cell line having resistance to a tyrosine kinase inhibitor (TKI) and gene expression in the original K562 cell line having no resistance to the tyrosine kinase inhibitor were compared using a DNA microarray (GenoCheck). The results are shown in FIG. 1.

As shown in FIG. 1, expression of Cobll1, ITGA4, ASB9, CD109 and ZNF462 genes increased rapidly in the cell line having resistance to the tyrosine kinase inhibitor (TKI). In addition, in experiments performed using samples isolated from patients, it was shown that the expression patterns of genes known to increase resistance to tyrosine kinase inhibitors changed when chronic myeloid leukemia (CML) transformed to an acute phase, and that expression of the genes increased in patients in whom chronic myeloid leukemia has progressed to acute leukemia (AML or ALL). Thus, it was found that resistance to the tyrosine kinase inhibitor appeared at a high rate. From these results, it could be predicted that there would be some correlations between the progression of chronic myeloid leukemia to an acute phase and the mechanism of resistance to the tyrosine kinase inhibitor.

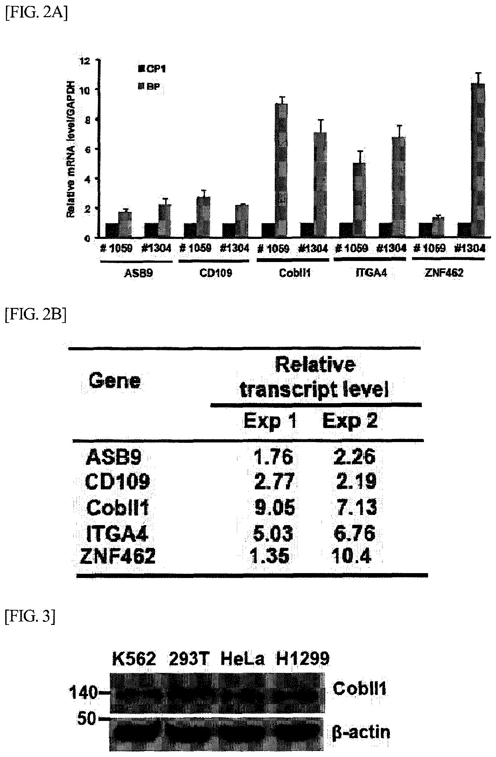

In addition, in order to reexamine whether the five genes influence the progression of chronic myeloid leukemia to an acute phase, the expression levels of the genes in the chronic phase and acute phase bone marrow (BM) from #1059 patient and #1304 patient were analyzed by quantitative real-time RT-PCR. For RT-PCR, RNA was extracted from cells using 1 mL of Trizol reagent and 0.2 mL of chloroform, and 0.5 mL of isopropyl alcohol was added to the extracted RNA and centrifuged at 12,000xg for 15 minutes to precipitate the RNA. The precipitated RNA was separated. According to a predetermined protocol, the separated RNA was reverse-transcribed using a Reverse Transcription System (Promega), and then subjected to RT-PCR using TaqMan® universal PCR master mix under the following conditions: 2 min at 50 °C, and then 45 cycles, each consisting of 20 sec at 95°C, 3 sec at 95°C, and 30 sec at 60°C. The TaqMan Probe and Cobll1 and GAPDH (control) primers used were purchased from Applied Biosystems. The results are shown in FIG. 2.

As shown in FIG. 2, expression of the Cobll1 gene among the five genes increased about 8-fold in the acute phase. The Cobll1 gene is known to act as a negative regulator of apoptosis and show resistance to low concentrations of insulin. In addition to such known actions, from the results as described above, it could be seen that the Cobll1 gene would play an important role in the proliferation and survival of cancer cells in the progression of chronic myeloid leukemia to an acute phase.

### Example 2: Production of Anti-Cobll1 Antibody

In order to examine the role of Cordon-bleu protein-like 1 (Cobll1) protein (SEQ ID NO: 1) in chronic myeloid leukemia, an anti-Cobll1 antibody was produced. For antibody production, the C-terminus (amino acid residues 701 to 100 in the amino acid sequence of SEQ ID NO: 2) of the Cobll1 protein was inserted into a GST-vector, and the vector was used as an antigen and injected first into a rabbit by subcutaneous injection. After 2 weeks, the vector was injected second into the rabbit, and on day 55, blood was taken from the rabbit From the taken rabbit blood, a rabbit-anu-Cobll1 antibody was separated using the Aminolink Coupling Gel Column Kit according to a predetermined protocol. The results are shown in FIG. 3.

As shown in FIG. 3, the antibody that binds to the Cobll1 protein was normally produced.

### Example 3: Analysis of Correlation between Cobll1 Protein and Progression of Chronic Myeloid Leukemia to Acute Phase

### 3.1: Analysis of Correlation between Cobll1 Protein and Progression of Chronic Myeloid Leukemia to Acute Phase

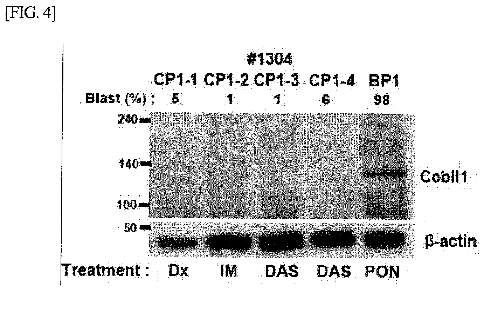

In order to examine whether the Cobll1 protein is involved in the progression of chronic myeloid leukemia to an acute phase by use of the anti-Cobll1 antibody produced in the same manner as described in Example 2, Western blotting was performed using bone marrow cells of the acute-phase sample isolated from #1304 patient. Briefly, cells were washed twice with cold PBS (phosphate buffered saline) buffer, and lysed in RIPA buffer, and protein was isolated from the cells. For subsequent experiments, the amount of the isolated protein was measured by a BCA assay. Furthermore, 20 µg of the protein obtained from each cell line was separated using 8-10% SDS-polyacrylamide gel and transferred to a PVDF membrane by electroblotting. The PVDP membrane having the protein transferred thereto was treated with 5% skim milk powder at room temperature for 1 hour, and then incubated with primary antibody against each of Coblll and beta-actin (control) at 4°C for 16 hours. The PVDP membrane incubated with the antibody was washed three times with TBST buffer to remove unbound primary antibody, and was further incubated with HPR-conjugated secondary antibody at room temperature for 1 hour. After completion of the reaction, the membrane was washed with TBST buffer to completely remove the secondary antibody, after which it was treated with ECL solution, incubated for 3 minutes, and then developed with Kodak X-OMAT AR Film. Among the antibodies used for Western blotting, Anti-Myc was purchased from Roche, and anti-beta-actin and HRP-conjugated secondary antibody were purchased from Sigma-Aldrich. These antibodies were used after dilution at 1:2000. In addition, the percentage of leukemic blasts in the bone marrow from each patient was determined. The results are shown in FIG. 4.

As shown in FIG. 4, expression of the Coblll protein increased markedly in the acute phase. These results suggest that the Coblll protein is involved in the progression of chronic myeloid leukemia to an acute phase.

### 3.2: Analysis of Patient-Dependent Difference in Correlation between Coblll Protein and Progression of Chronic Myeloid Leukemia to Acute Phase

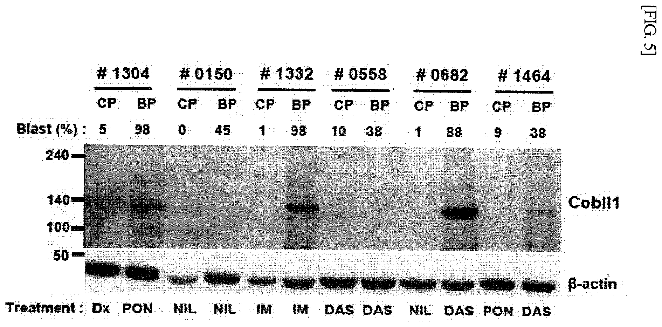

In order to examine whether there is a difference in expression of the Coblll protein between patients, Western blotting was performed on six patients in the same manner as described in Example 3.1. The results are shown in FIG. 5.

As shown in FIG. 5, expression of the Coblll protein increased in only four of the six patients, suggesting that expression of the Cobll1 protein is associated with the number of leukemic blasts.

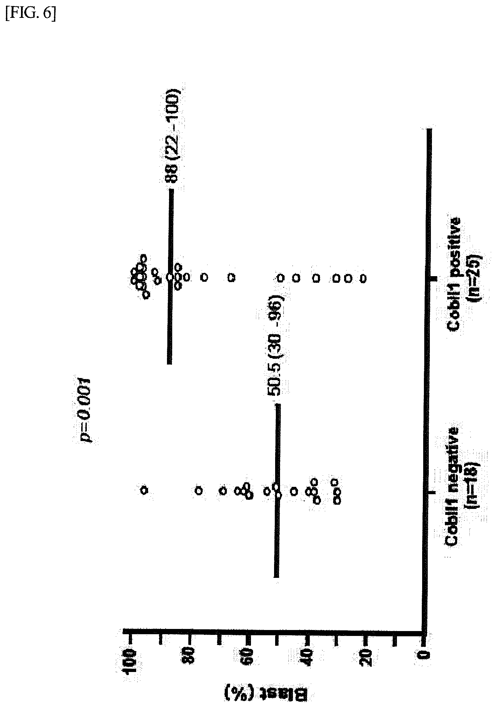

To confirm this again, on 43 patients who have progressed to an acute phase during treatment with various tyrosine kinase inhibitors (TKIs) after diagnosed as chronic myeloid leukemia, the expression level of the Cobll1 protein and the amount of leukemic blasts were measured. The results are shown in FIG. 6.

As shown in FIG. 6, expression of the Coblll protein increased in 25 of the 43 patients. In addition, it was shown that when the percentage of leukemic blasts was 60% or less, expression of the Cobll1 protein did not increase with high probability, and when the percentage of leukemic blasts was 70% or more, expression of the Cobll1 protein increased with high probability. These results suggest that the number of leukemic blasts in peripheral blood and bone marrow and the expression of the Cobll1 protein in peripheral blood and bone marrow are associated directly with the progression of chronic myeloid leukemia.

### 3.3: Analysis of Difference in Expression Level of Cobll1 Protein between Different Samples

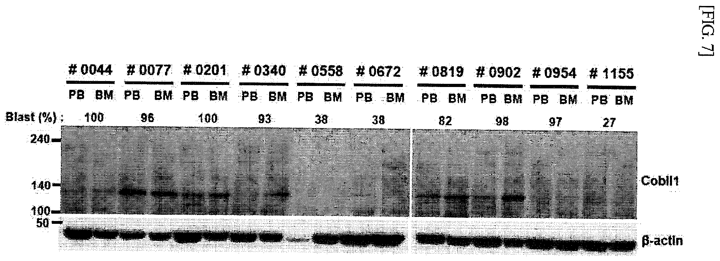

In order to examine whether the expression level of Cobll1 protein measured differs depending on the kind of sample, samples were taken from the peripheral blood and bone marrow of 10 patients, and the expression level of the Cobll1 protein in each of the samples was measured. The results are shown in FIG. 7.

As shown in FIG. 7, the results measured in the peripheral blood were the same as those measured in the bone marrow. These results suggest that the progression of chronic myeloid leukemia to an acute phase can be predicted using not only a bone marrow sample but also a peripheral blood sample.

### 3.4: Analysis of Difference in Expression Level of Cobll1 Protein between Progression Stages of Chronic Myeloid Leukemia

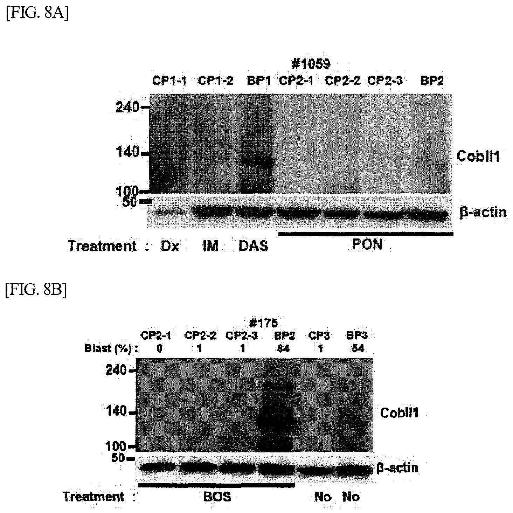

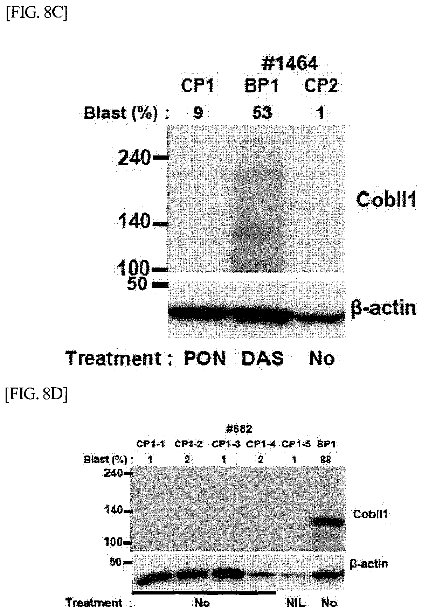

In order to specifically examine the expression level of the Cobll1 protein according to the progression stage of chronic myeloid leukemia, the expression of the Cobll1 protein in each progression stage of chronic myeloid leukemia in four patient with recurrent myeloid leukemia was analyzed in the same manner as described in Example 3.1. The results are shown in FIG. 8.

As shown in FIG. 8, expression of the Cobll1 protein increased rapidly according to the progression stage of chronic myeloid leukemia. Particularly, it was shown that expression of the Cobll1 protein increased rapidly in the initial stage of progression to an acute phase. These results suggest that the Cobll1 protein plays an important role in the progression of chronic myeloid leukemia.

### Example 4: Analysis of Correlation between Cobll1 Protein and BCR-ABL1

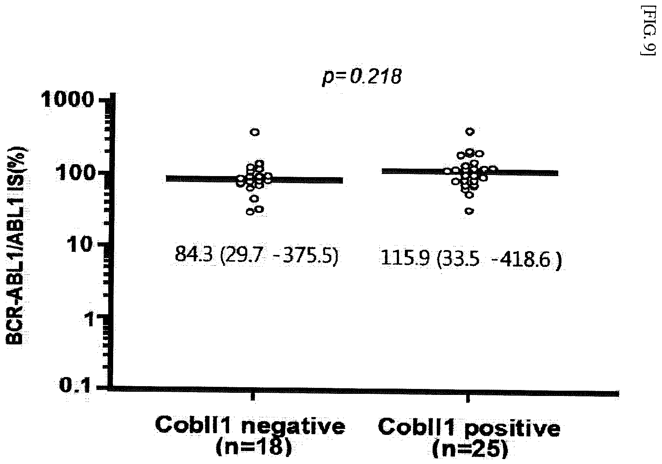

In order to examine the correlation between the BCR-ABL1 protein and the Cobll1 protein in the progression of chronic myeloid leukemia, the expression level of each of the proteins in the acute phase was measured in the same manner as described in Example 3.1. The results are shown in FIG. 9.

As shown in FIG. 9, expression of the BCR-ABL1 protein had no correlation with expression of the Cobll1 protein. These results suggest that the BCR-ABL1 protein has no correlation with the progression of chronic myeloid leukemia to an acute phase, in which the Cobll1 protein is involved.

### Example 5: Examination of Medical Information

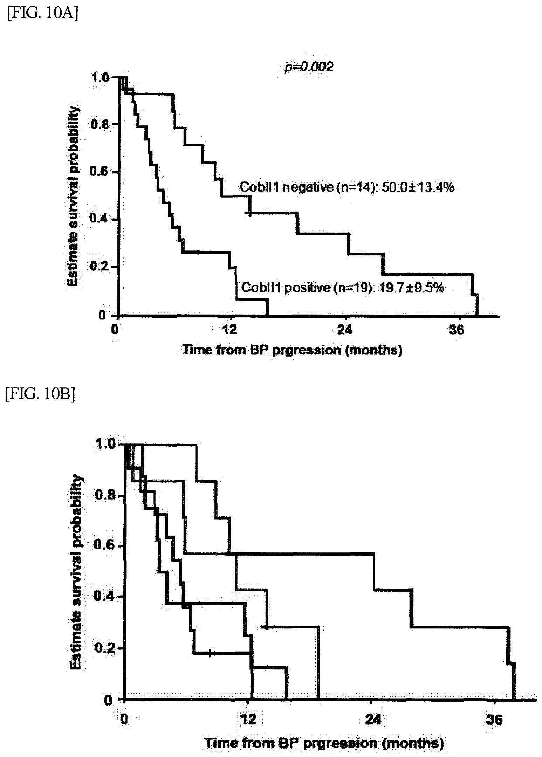

In order to examine other medical information on the progression of chronic myeloid leukemia to an acute phase, in which the Cobll1 protein is involved, examination was performed of various medical information, including the subject's age and sex, the kind of acute phase, BCR-ABL mutation, survival time and the like for the 43 samples described in Example 3.2. The results are shown in FIG. 10 and Tables 1 and 2 below.

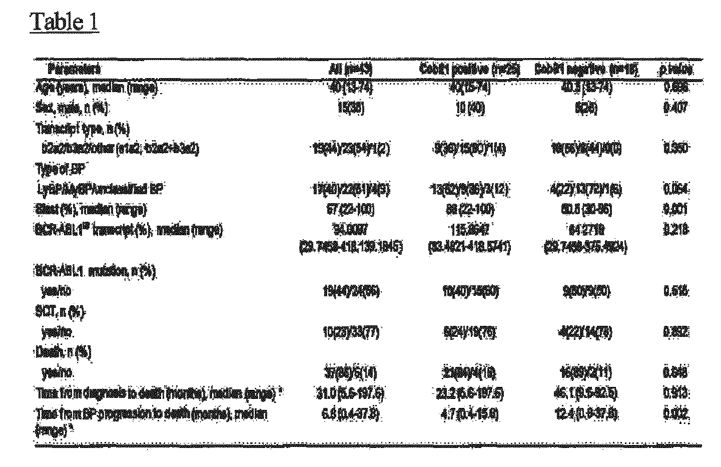

As shown in FIG. 10A and Table 1 below, it was confirmed again that the expression level of theCobll1 protein did differ depending on the percentage ofleukemic blasts and that there was asignificant difference in time to death after progression to the acute phase. In addition, it was shown that patients who showed increased expression of the Cobll1 protein survived for 4.7 months on average after progression to the acute phase, and that the survival time of patients who showed no increased expression of the Cobll1 protein increased by 12.4 months on average.

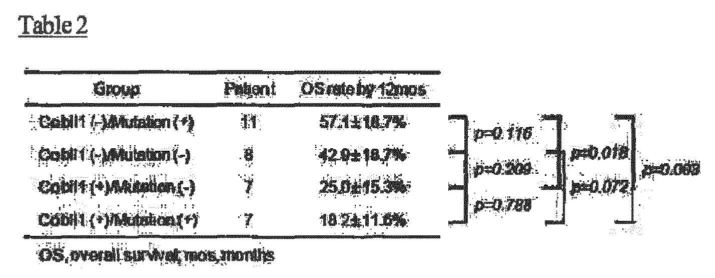

In addition, as shown in FIG. 10B and Table 2 above, increased expression of the Cobll1 gene more influenced the prognosis of chronic myeloid leukemia patients after progression to the acute phase than BCR-ABL1 mutation.

These results suggest that the prognosis of chronic myeloid leukemia patients who have progressed to an acute phase can be determined by analyzing expression of the Cobll1 protein and also that the Cobll1 protein in acute-phase cells plays an important role in transformation into an acute phase and resistance to tyrosine kinase inhibitors.

In addition, these results indicate that the prognosis of survival after transformation into an acute phase can be predicted by measuring expression of the Cobll1 protein in biological samples isolated from chronic myeloid leukemia patients.

### Example 6: Examination of Role of Cobll1 Protein

### 6.1: Analysis of Difference in Cobll1 Protein Expression between Cell Lines

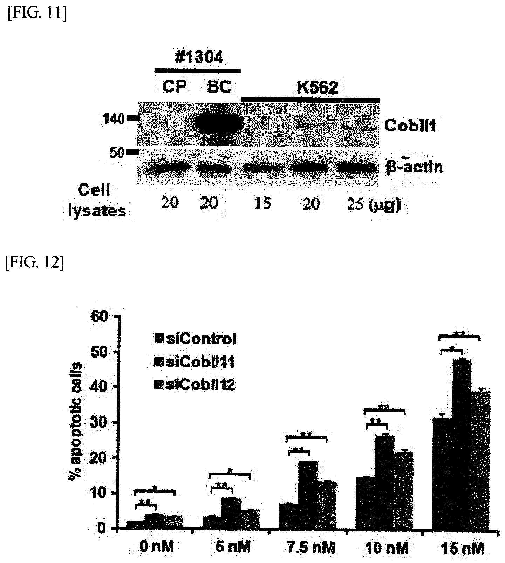

In order to examine the role of the Cobll1 protein in chronic myeloid leukemia, the expression levels of the Cobll1 protein in chronic-phase cells, acute-phase cells and the K562 cell line were first analyzed in the same manner as described in Example 3.1. For experiments, each type of cell was cultured in RPMI supplemented with 10% fetal bovine serum (FBS). The results are shown in FIG. 11.

As shown in FIG. 11, expression of the Cobll1 protein did not increase in the K562 cell line and the chronic-phase cells. In a subsequent experiment, the role of the Cobll1 protein was examined using the K562 cell line.

### 6.2: Analysis of Correlation between Cobll1 Protein and Drug Resistance

In order to examine the effect of the Cobll1 protein on drug resistance, Cobll1 expression in the K562 cell line was inhibited using an siRNA against Cobll1. The siRNA used was purchased from IDT (siRNA1 sense (SEQ ID NO. 3): 5'-CUAUGAUGGACUUGUUGAUUU-3'; siRNA1 antisense (SEQ ID NO. 4): 5'-AUCAACAAGUCCAUCAUAGUU-3'; siRNA2 sense (SEQ ID NO. 5): 5'-GACUCAUUCUGUAAAUAAAUU-3'; siRNA2 antisense (SEQ ID NO: 6): 5'-UUUAUUUACAGAAUGAGUCUU-3'; and control siRNA (5'-TTCAATAAATTCTTGAGGTTT-3'). Furthermore, the cells with inhibited expression of the Coblll protein were treated with the second-generation tyrosine kinase inhibitor nilotimb, and apoptosis of the treated cells was examined using the FITC Annexin V Apoptosis Detection Kit I (BD Science) according to a predetermined protocol. The results are shown in FIG. 12.

As shown in FIG. 12, nilotinib-induced apoptosis of the cells with inhibited expression of the Coblll protein increased. This suggests that when expression of the Cobll1 protein is inhibited, sensitivity to nilotinib increases.

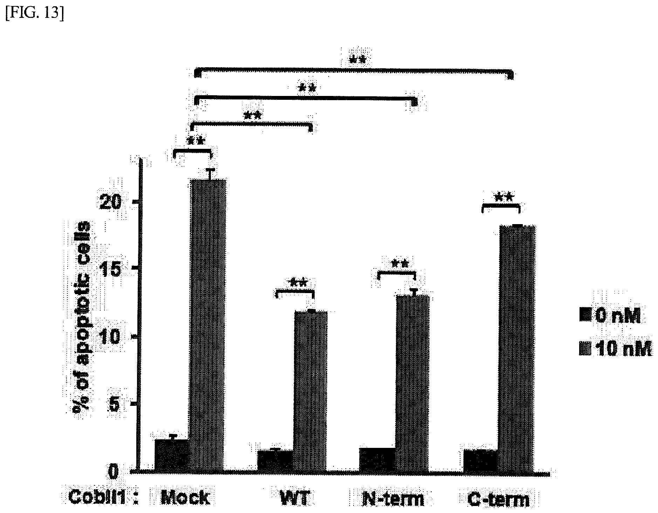

In addition, in order to further examine the correlation between expression of the Cobll1 protein and drug resistance, the degree of nilotinib-induced apoptosis was analyzed using the full-length Coblll protein (SEQ ID NO: 1), the N-terminus of the Cobll1 protein (amino acid residues 1 to 700), and the C-terminus of the Cobll1 protein (amino acid residues 701 to 1128). The results are shown in FIG. 13.

As shown in FIG. 13, nilotinib-induced apoptosis of the cells with overexpression of the Cobll1 protein decreased, and the N-terminus of the Cobll1 protein showed greater resistance to apoptosis than the C-terminus. These results suggest that the Cobll1 protein plays an important role in resistance to tyrosine kinase inhibitor drugs, and particularly, the N-terminus of the Cobll1 protein plays an important role in resistance to the drugs.

### 6.3: Evaluation of the Effect of Cobll1 Protein on Cell Growth and Transformation into Leukemic Cells

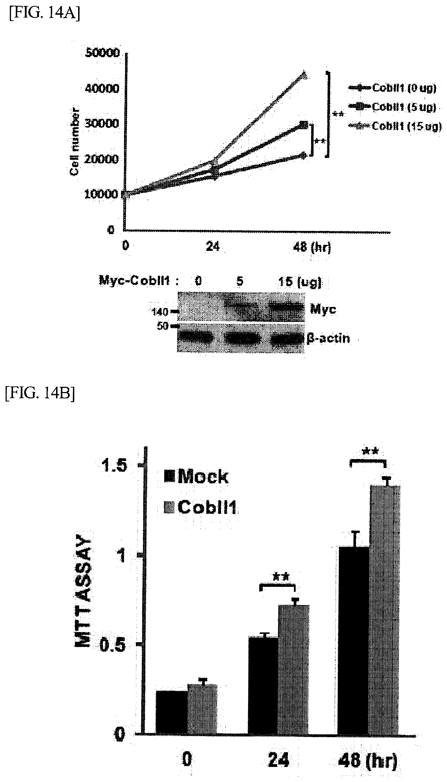

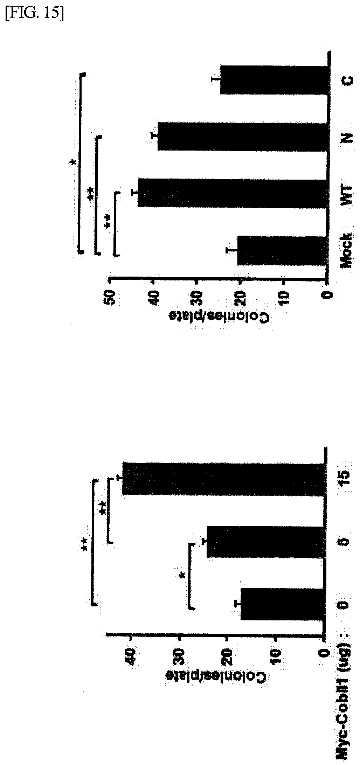

In order to evaluate the effect of the Cobll1 protein on cell growth and transformation into chronic myeloid leukemia cells, the Cobll1 protein in the K562 cell line was overexpressed, and then the degree of proliferation of the cells was analyzed. The Cobll1 plasmid (Clone ID: 30345650) used to overexpress the Cobll1 protein was purchased from Thermo Scientific. 20 µg of the plasmid DNA purchased for transformation was transfected into cells together with R buffer by electroporation, and the transfected cells were incubated for 24 or 48 hours, and then used in experiments. The degree of proliferation of the cells was analyzed by 3-(4,5-dimethylthiazol-2-yl)-2,5-diphenyltetrazolium bromide (MTT) assay (Promega) according to a predetermined protocol. In addition, the degree of transformation into chronic myeloid leukemia cells was analyzed by soft agar assay. The results are shown in FIGS. 14 and 15.

As shown in FIG. 14, cell proliferation increased depending on the concentration of the Cobll1 protein.

In addition, as shown in FIG. 15, transformation into chronicmyeloid leukemia cells significantly increased depending on the concentration of the Cobll1 protein. Furthermore, it could be seen that the N-terminus of the Cobll1 protein played an important role in transformation into chronic myeloid leukemia cells.

These results suggest that the Cobll1 protein acts as an oncoprotein and plays an important role in cell growth and transformation into chronic myeloid leukemia cells.

From the results as described above, it could be seen that expression of the Cobll1 protein plays an important role in the progression of chronic myeloid leukemia to an acute stage and in increasing resistance to drugs. This suggests that inhibition of expression of the Cobll1 protein can reduce the progression of chronic myeloid leukemia and resistance to drugs, and thus can be used for the treatment of chronic myeloid leukemia, and can also be used to return chronic myeloid leukemia patients, who have progressed to an acute phase, to a chronic phase. In addition, through additional signaling experiments, the present inventors have found that the Cobll1 protein activates NF-kB to increase the stability of IKKr, thereby ultimately increasing the progression of chronic myeloid leukemia and resistance to drugs.
## Claims

1. A method for (i) providing information on the progression of chronic myeloid leukemia to an acute phase, (ii) providing information on the prognosis of chronic myeloid leukemia, (iii) providing information on the resistance of a chronic myeloid leukemia patient to a tyrosine kinase inhibitor, the method comprising:
- measuring the expression level of Cordon-bleu protein-like 1 (Cobll1) in a patient's biological samples, wherein the biological sample is one or more selected from the group consisting of blood, plasma, serum and bone marrow,
- comparing the obtained values with those of a control group,
wherein, if the measured Cobll1 value is increased with respect to the control: (i) chronic myeloid leukemia has progressed to the acute phase, (ii) the prognosis is negative and survival is predicted to be short, (iii) the patient is resistant to tyrosine kinase inhibitors.

2. The method of claim 1, wherein the tyrosine kinase inhibitor is imatinib or nilotinib.

3. The method of claim 1, wherein a method for measuring the expression level of Coblll is one or more selected from the group consisting of radioimmunoassay, radioimmunoprecipitation assay, immunoprecipitation assay, ELISA (enzyme-linked immunosorbentassay), sandwich assay, real-time PCR, RT-PCR, and Western blotting.

4. Use of an antibody, an aptamer or a ligand specific for Cordon-bleu protein-like 1 (Cobll1), or a probe set complementary to a Cobll1 nucleic acid sequence, or a primer set complementary to a Cobll1 nucleic acid sequence, for:
(i) providing information on the progression of chronic myeloid leukemia to an acute phase, (ii) providing information on the prognosis of chronic myeloid leukemia, or (iii) providing information on the resistance of a chronic myeloid leukemia patient to a tyrosine kinase inhibitor.

5. The use of claim 4, wherein the antibody is produced using the amino acid sequence of SEQ ID NO: 2 as an antigen.

6. The use of claim 4, wherein said molecular tools are in the form of a kit.

7. A pharmaeutical composition comprising an oligonucleotide that binds to, and inhibits the expression of Cordon-bleu protein-like 1 (Cobll1) mRNA for use in treating chronic myeloid leukemia.

8. The pharmaceutical composition of claim 7, wherein the oligonucleotide is siRNA (silencing RNA), miRNA (miRNA) or shRNA (short hairpin RNA).

9. The pharmaceutical composition of claim 8, wherein the siRNA comprises one or more of the nucleotide sequences of SEQ ID NOs: 3 to 6.

## Patentansprüche

1. Verfahren zum (i) Bereitstellen von Information über das Fortschreiten von chronischer myeloischer Leukämie in eine akute Phase, (ii) Bereitstellen von Information über die Prognose von chronischer myeloischer Leukämie, (iii) Bereitstellen von Information über die Resistenz von einem Chronische-myeloische-Leukämie-Patienten gegenüber einem Tyrosinkinase-Inhibitor, wobei das Verfahren umfasst:
- Messen des Expressionsniveaus von Cordon-bleu protein-like 1 (Cobll1) in einer biologischen Probe eines Patienten, wobei die biologische Probe eines oder mehrere ist, das/die ausgewählt ist/sind aus der Gruppe, bestehend aus Blut, Plasma, Serum und Knochenmark,
- Vergleichen der erhaltenen Werte mit denen einer Kontrollgruppe,
wobei, wenn der gemessene Cobll1-Wert in Bezug auf die Kontrolle erhöht ist: (i) chronische myeloische Leukämie in die akute Phase fortgeschritten ist, (ii) die Prognose negativ ist und vorhergesagt wird, dass ein Überleben kurz ist, (iii) der Patient resistent gegenüber Tyrosinkinase-Inhibitoren ist.

2. Verfahren nach Anspruch 1, wobei der Tyrosinkinase-Inhibitor Imatinib oder Nilotinib ist.

3. Verfahren nach Anspruch 1, wobei ein Verfahren zum Messen des Expressionsniveaus von Coblll eines oder mehrere ist, das/die ausgewählt ist/sind aus der Gruppe, bestehend aus Radioimmunassay, Radioimmunpräzipitationsassay, Immunpräzipitationsassay, ELISA (enzymgekoppelter Immunadsorptionsassay), Sandwich-Assay, Echtzeit-PCR, RT-PCR und Western-Blotten.

4. Verwendung eines Antikörpers, eines Aptamers oder eines Liganden, der/das für Cordon-bleu protein-like 1 (Cobll1) spezifisch ist, oder eines Sondensets, das zu einer Cobll1-Nukleinsäuresequenz komplementär ist, oder eines Primersets, das zu einer Cobll1-Nukleinsäuresequenz komplementär ist, zum:
(i) Bereitstellen von Information über das Fortschreiten von chronischer myeloischer Leukämie in eine akute Phase, (ii) Bereitstellen von Information über die Prognose von chronischer myeloischer Leukämie oder (iii) Bereitstellen von Information über die Resistenz von einem Chronische-myeloische-Leukämie-Patienten gegenüber einem Tyrosinkinase-Inhibitor.

5. Verwendung nach Anspruch 4, wobei der Antikörper unter Verwendung der Aminosäuresequenz von SEQ ID NO: 2 als ein Antigen produziert wird.

6. Verwendung nach Anspruch 4, wobei die molekularen Werkzeuge in der Form eines Kits vorliegen.

7. Pharmazeutische Zusammensetzung, die ein Oligonukleotid umfasst, das an Cordon-bleu protein-like 1 (Cobll1) -mRNA bindet und die Expression davon inhibiert, zur Verwendung bei der Behandlung von chronischer myeloischer Leukämie.

8. Pharmazeutische Zusammensetzung nach Anspruch 7, wobei das Oligonukleotid siRNA (Silencing-RNA), miRNA (miRNA) oder shRNA (kurze Haarnadel-RNA) ist.

9. Pharmazeutische Zusammensetzung nach Anspruch 8, wobei die siRNA eine oder mehrere der Nukleotidsequenzen von SEQ ID NOs: 3 bis 6 umfasst.

## Revendications

1. Une méthode pour (i) fournir des informations sur la progression de la leucémie myéloïde chronique vers une phase aiguë, (ii) fournir des informations sur le pronostic de la leucémie myéloïde chronique, (iii) fournir des informations sur la résistance d'un patient atteint de leucémie myéloïde chronique à un inhibiteur de la tyrosine-kinase, la méthode comprenant :
- la mesure du niveau d'expression de la protéine Cordon bleu de type 1 (Cobll1) dans les échantillons biologiques d'un patient, où l'échantillon biologique est un ou plusieurs échantillons sélectionnés dans le groupe composé du sang, du plasma, du sérum et de la moelle osseuse
- la comparaison des valeurs obtenues avec celles d'un groupe contrôle
Où, si la valeur Cobll1 mesurée augmente par rapport au contrôle : (i) la leucémie myéloïde chronique a progressé en phase aigüe, (ii) le pronostic est négatif et la survie est limitée à court terme, (iii) le patient est résistant aux inhibiteurs de la tyrosine-kinase

2. La méthode de la revendication 1, où l'inhibiteur de la tyrosine-kinase est l'imatinib ou le nilotinib.

3. La méthode de la revendication 1, où une méthode pour mesurer le niveau d'expression de Coblll est une ou plusieurs méthodes sélectionnées dans le groupe composé du dosage radio-immunologique, du dosage par radio-immunoprécipitation, du dosage par immunoprécipitation, ELISA (essai immuno-enzymatique), du dosage sandwich, de la PCR en temps réel, de la RT-PCR et de la méthode de western blot.

4. L'utilisation d'un anticorps, un aptamère ou un ligand spécifique pour la protéine Cordon bleu de type 1 (Cobll1), ou d'un set de sondes complémentaire à une séquence d'acides nucléiques de Cobll1, ou un set d'amorces complémentaire à une séquence d'acides nucléiques de Cobll1, pour :
(i) Fournir des informations sur la progression de la leucémie myéloïde chronique vers une phase aigüe, (ii) fournir des informations sur le pronostic de la leucémie myéloïde chronique, ou (iii) fournir des informations sur la résistance d'un patient atteint de leucémie myéloïde chronique à un inhibiteur de la tyrosine-kinase.

5. L'utilisation de la revendication 4, où l'anticorps est produit en utilisant la séquence d'acides aminés SEQ ID NO : 2 en tant qu'antigène.

6. L'utilisation de la revendication 4, où ces outils moléculaires se présentent sous la forme de kit.

7. Une composition pharmaceutique comprenant un oligonucléotide qui se lie à, et inhibe l'expression de l'ARN messager de la protéine Cordon bleu de type 1 (Cobll1) pour son utilisation dans le traitement de la leucémie myéloïde chronique.

8. La composition pharmaceutique de la revendication 7, où l'oligonucléotide est un siARN(ARN interférent), le miARN (micro-ARN) ou le shARN (petit ARN en épingle à cheveux).

9. La composition pharmaceutique de la revendication 8, où le siARN comprend une ou plusieurs des séquences nucléotidiques SEQ ID NO : 3 à 6.


<110> CATHOLIC UNIVERSITY INDUSTRY ACADEMIC COOPERATION FOUNDATION SungKyunKwan University's Research & Business Foundation
<120> Method for providing the information for chronic myeloid leukemia
<130> OPB163168PCT
<150> KR 10-2015-0113704
   <151> 2015-08-12
<160> 6
<170> KoPatentIn 3.0
<210> 1
   <211> 1128
   <212> PRT
   <213> Artificial Sequence
<220>
   <223> Cobll1 protein
<400> 1
<210> 2
   <211> 300
   <212> PRT
   <213> Artificial Sequence
<220>
   <223> Cobll1 antigen
<400> 2
   Leu Lys Asn Tyr Pro Leu Tyr Arg Gln Asp Tyr Asn Pro Lys Pro Lys
   1               5                   10                  15
   Pro Ser Asn Glu Ile Thr Arg Glu Tyr Ile Pro Lys Ile Gly Met Thr
               20                  25                  30
   Thr Tyr Lys Ile Val Pro Pro Lys Ser Leu Glu Ile Ser Lys Asp Trp
           35                  40                  45
   Gln Ser Glu Thr Ile Glu Tyr Lys Asp Asp Gln Asp Met His Ala Leu
       50                  55                  60
   Gly Lys Lys His Thr His Glu Asn Val Lys Glu Thr Ala Ile Gln Thr
   65                  70                  75                  80
   Glu Asp Ser Ala Ile Ser Glu Ser Pro Glu Glu Pro Leu Pro Asn Leu
                   85                  90                  95
   Lys Pro Lys Pro Asn Leu Arg Thr Glu His Gln Val Pro Ser Ser Val
               100                 105                 110
   Ser Ser Pro Asp Asp Ala Met Val Ser Pro Leu Lys Pro Ala Pro Lys
           115                 120                 125
   Met Thr Arg Asp Thr Gly Thr Ala Pro Phe Ala Pro Asn Leu Glu Glu
       130                 135                 140
   Ile Asn Asn Ile Leu Glu Ser Lys Phe Lys Ser Arg Ala Ser Asn Ala
   145                 150                 155                 160
   Gln Ala Lys Pro Ser Ser Phe Phe Leu Gln Met Gln Lys Arg Val Ser
                   165                 170                 175
   Gly His Tyr Val Thr Ser Ala Ala Ala Lys Ser Val His Ala Ala Pro
               180                 185                 190
   Asn Pro Ala Pro Lys Glu Leu Thr Asn Lys Glu Ala Glu Arg Asp Met
           195                 200                 205
   Leu Pro Ser Pro Glu Gln Thr Leu Ser Pro Leu Ser Lys Met Pro His
       210                 215                 220
   Ser Val Pro Gln Pro Leu Val Glu Lys Thr Asp Asp Asp Val Ile Gly
   225                 230                 235                 240
   Gln Ala Pro Ala Glu Ala Ser Pro Pro Pro Ile Ala Pro Lys Pro Val
                   245                 250                 255
   Thr Ile Pro Ala Ser Gln Val Ser Thr Gln Asn Leu Lys Thr Leu Glu
               260                 265                 270
   Thr Phe Gly Ala Pro Arg Pro Tyr Ser Ser Ser Gly Pro Ser Pro Phe
           275                 280                 285
   Ala Leu Ala Val Val Lys Arg Ser Gln Ser Phe Ser
       290                 295                 300
<210> 3
   <211> 21
   <212> RNA
   <213> Artificial Sequence
<220>
   <223> si Cobll1
<400> 3
   cuaugaugga cuuguugauu u 21
<210> 4
   <211> 21
   <212> RNA
   <213> Artificial Sequence
<220>
   <223> si Cobll1
<400> 4
   aucaacaagu ccaucauagu u 21
<210> 5
   <211> 21
   <212> RNA
   <213> Artificial Sequence
<220>
   <223> si Cobll1
<400> 5
   gacucauucu guaaauaaau u 21
<210> 6
   <211> 21
   <212> RNA
   <213> Artificial Sequence
<220>
   <223> si Cobll1
<400> 6
   uuuauuuaca gaaugagucu u 21